# EUROPEAN PATENT APPLICATION

(11) **EP 2 201 979 A1**
(43) Date of publication of application: **30.06.2010**
(21) Application number: 08396019.5
(22) Date of filing: 23.12.2008
(51) Int. Cl.: A61M 16/01

(54) **Arrangement and method for supplying breathing gas for respiration**

(71) Applicant: General Electric Company, Schenectady, NY 12345 (US)
(72) Inventor: Lähde, Niina, 02760 Espoo (FI); Heinonen, Erkki, 00750 Helsinki (FI)
(74) Representative: Kanerva, Arto

(57) **Abstract**

An arrangement for supplying a breathing gas for a respiration is disclosed herein. The arrangement includes a reciprocating unit (6) receiving an inspiration gas flow adapted to control respiratory movements and a gas mixer (3) for supplying a fresh gas flow. The arrangement also includes a breathing circuit (2) for conducting an expiration gas flow to the reciprocating unit (6) and for conducting the fresh gas flow from the gas mixer (3) for the respiration and for conducting the gas flow from the reciprocating unit (6) for an inspiration, the breathing circuit comprising a CO2 remover (20) for removing carbon dioxide from the gas. The arrangement also includes a first bypass passage (51) bypassing the reciprocating unit (6) and connecting the breathing circuit (2) to the inspiration gas flow upstream the reciprocating unit (6). A corresponding method for supplying a breathing gas for a respiration is also provided.

## Description

### BACKGROUND OF THE INVENTION

This disclosure relates generally an arrangement and method for supplying a breathing gas for a respiration.

Hospital efficiency requirements get more demanding, more patients have to be taken through a surgery process in a shorter time and less costs. In spite of all, a patient safety is a primary requirement. Anesthesia machines are used for anesthetizing and ventilating patients in hospital operating rooms, intensive care units or other patient care places.

A patient breathing circuit can be a re-breathing circuit or an open circuit. The re-breathing circuit with a CO2 absorber is used when volatile anesthetic or NO2, or both, exist in anesthesia. Instead the open circuit is used with an intravenous (IV) anesthesia.

Inhalation anesthesia is thus delivered using the re-breathing circuit comprising an inspiratory limb through which a patient gets an inspired breathing gas from a ventilator, an expiratory limb carrying an exhaled gas back to the ventilator, a Y-piece connecting the inspiratory and expiratory limbs to a further patient limb providing a gas communication pathway to patient lungs. An expired gas comprises a lot of expensive and environment-hostile anesthesia gases. The re-breathing circuit is used to return the expired gas to next inspiration. Before doing this, the gas must be cleared out from the carbon dioxide extracted from the patient lungs as waste product of a patient metabolism. The clearance is done in a CO2 absorber.

Traditional anesthesia ventilator comprises a bellows-in-bottle, which separates a ventilator drive gas from the circulating breathing gas. For the inspiration, outside of the bellows is pressurized with ventilator drive gas. This squeezes the bellows forcing the breathing gas within the bellows to flow towards the patient lungs. During the expiration, the drive gas pressure is released and the gas pressurized in the patient lungs flows out filling the bellows. Breathing gas is supplied to the re-breathing circuit as continuous flow using a fresh gas line. Once the bellows is filled, further gas flow to the circuit from the patient and the fresh gas line increases the circuit pressure that opens an over-pressure bleed valve for a removal of this further gas to a scavenging system.

In a new re-breathing circuit drive gas - a breathing gas separation is done using a long gas channel where a gas column is moving back-and-forth during the course of the inspiration and the expiration. The re-breathing circuit saves anesthesia gases, and hence lowers the hospitals anesthesia expenses, and decreases environment effects. The CO2 absorption and the re-breathing circuit are not needed when an anesthetic is not breathable, but e.g. an intravenous-drug. The intravenous-anesthesia is gaining popularity and optimal solution is needed there too. The open breathing circuit is more simple solution than the re-breathing circuit, but it cannot be used with the inhalation anesthesia. It is cheaper and easier, to utilize the open breathing circuit in the IV-anesthesia. But sometimes the IV-anesthesia needs to exchange to inhalation anesthesia during the case. In this case the re-breathing circuit is needed because of the anesthesia gases. In state-of the art devices such switchover is not possible and therefore IV anesthesia is delivered using non-optimal equipment. Current anesthesia solution is the re-breathing circuit whereas the open circuit is used in an intensive care ventilation.

### BRIEF DESCRIPTION OF THE INVENTION

The above-mentioned shortcomings, disadvantages and problems are addressed herein which will be understood by reading and understanding the following specification.
In an embodiment, an arrangement for supplying a breathing gas for a respiration includes a reciprocating unit receiving an inspiration gas flow adapted to control respiratory movements and a gas mixer for supplying a fresh gas flow. The arrangement for supplying a breathing gas for a respiration also includes a breathing circuit for conducting an expiration gas flow to the reciprocating unit and for conducting the fresh gas flow from the gas mixer for the respiration and for conducting the gas flow from the reciprocating unit for an inspiration, the breathing circuit comprising a CO2 remover for removing carbon dioxide from the gas. The arrangement for supplying a breathing gas for a respiration further includes a first bypass passage bypassing the reciprocating unit and connecting the breathing circuit to the inspiration gas flow upstream the reciprocating unit.

In another embodiment, a method for supplying a breathing gas for a respiration includes supplying an inspiration gas flow to a reciprocating unit to control respiratory movements and supplying a fresh gas flow to a breathing circuit. The method for supplying a breathing gas for a respiration also includes conducting along a breathing circuit an expiration gas flow to the reciprocating unit and the fresh gas flow for the respiration and a gas flow from the reciprocating unit for inspiration, and removing carbon dioxide from the gas flowing in the breathing circuit. The method for supplying a breathing gas for a respiration further includes an option to supply the inspiration gas flow to the breathing circuit bypassing the reciprocating unit.

In yet another embodiment an arrangement for supplying a breathing gas for a respiration includes a ventilator having a reciprocating unit receiving an inspiration gas flow along a first canal and discharging an expiration gas flow along a second canal, the reciprocating unit being adapted to control respiratory movements. The arrangement for supplying a breathing gas for a respiration also includes a gas mixer for supplying a fresh gas flow for a respiration and a breathing circuit for conducting an expiration gas flow to the ventilator and for conducting the fresh gas flow from the gas mixer for the respiration and for conducting the gas flow from the ventilator for the inspiration, the breathing circuit comprising a CO2 remover for removing carbon dioxide from the gas. The arrangement for supplying a breathing gas for a respiration further includes a first bypass passage connecting the first canal and the breathing circuit and further comprising a second bypass passage connecting the breathing circuit and the second canal wherein both the first bypass passage and the second bypass passage are adapted to bypass the reciprocating unit.

Various other features, objects, and advantages of the invention will be made apparent to those skilled in art from the accompanying drawings and detailed description thereof.

### BRIEF DESCRIPTION OF THE DRAWINGS

Figure 1 is a schematic view of a system in accordance with an embodiment;

Figure 2 is a schematic view of a system in accordance with another embodiment;

Figure 3 shows a selector valve in a first position useful for embodiments shown in Figure 1 or 2;

Figure 4 shows the selector valve of Figure 3 in a second position;

Figure 5 shows another selector valve in a first position useful for embodiments shown in Figure 1 or 2; and

Figure 6 shows the selector valve of Figure 5 in a second position.

### DETAILED DESCRIPTION OF THE INVENTION

Specific embodiments are explained in the following detailed description making a reference to accompanying drawings. These detailed embodiments can naturally be modified and should not limit the scope of the invention as set fort in the claims.

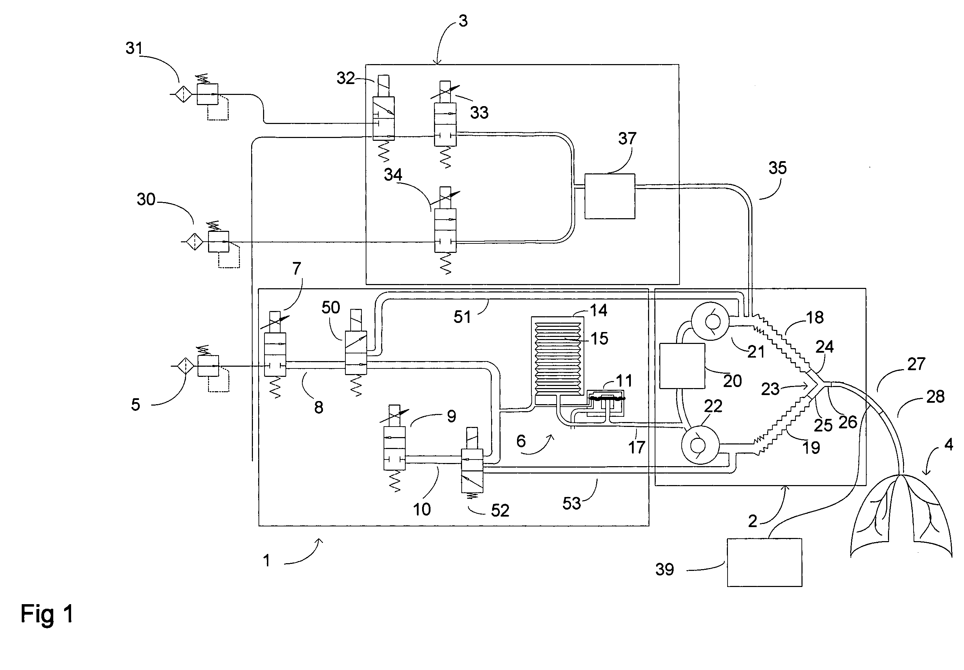

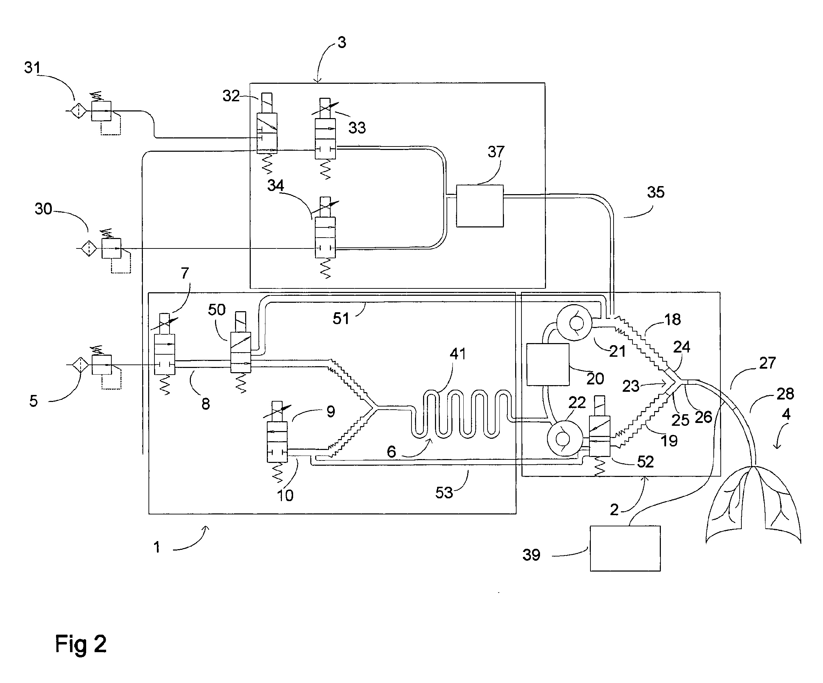

Figures 1 and 2 shows an arrangement for exchanging between a re-breathing circuit and open circuit modes in an anesthesia system. The anesthesia system comprises a ventilator 1, a breathing circuit 2 and a fresh gas mixer 3. A subject 4 is connected to the breathing circuit 2 by means of an endotracheal tube 28.

In Figure 1 the ventilator 1 is connected to a gas supply 5, which is typically pressurized air or sometimes also oxygen. The ventilator 1 comprises a reciprocating unit 6 for compressing gas towards lungs of the subject to facilitate the inspiration, a flow control valve 7 to control the inspired gas flow from the gas supply 5 along a first canal 8 towards the reciprocating unit 6. Further the ventilator 1 comprises an expiration valve 9 used to control the expired gas flow rate through a second canal 10 releasing the gas of the ventilator 1 or the reciprocating unit 6 when the subject 4 is expiring. The ventilator 1 can also comprise a scavenging valve 11 allowing an extra expired gas of the subject 4 to leave the breathing circuit 2. The ventilator 1 may be equipped with a gas supply selection (not shown) that can be used to switch the ventilator gas flow for the inspiration either manually or automatically e.g. in case the used gas gets un-pressurized. The reciprocating unit 6 shown in Figure 1 comprises a bottle 14 and a bellows 15 within the bottle 14 for controlling respiratory movements of the subject's lungs.

When ventilating the subject, the expiration valve 9 is closed and the flow control valve 7 is opened for the inspiration flow. This flow fills the bottle 14 making the bellows 15 to push down pushing the gas within the bellows further towards the subject 4. During the expiration the flow control valve 7 is closed and the expiration valve 9 is opened to control the expiration flow and pressure. The gas pressurized in the bottle 14 is released allowing the gas from lungs to fill the bellows 15 up. When the bellows 15 is filled, it hits the top of the bottle 14, and the further gas flow into the system increases the pressure within the bellows 15. When this pressure exceeds the bottle pressure, the scavenging valve 11 will open allowing the further gas flow to scavenging system.

The ventilator 1 is connected to the breathing circuit 2 such as a re-breathing circuit by means of the ventilator tube 17 for both inspired and expired gas flows. The breathing circuit 2 comprises an inspiration tube 18 for inspired gas, an expiration tube 19 for expired gas, a CO2 remover 20 such as CO2 absorber to remove or absorb carbon dioxide from the exhaled gas coming from the subject 4, a first one-way valve 21 for inspired gas in the inspiration tube 18, a second one-way valve 22 for expired gas in the expiration tube 19, a branching unit 23 such as a Y-piece having at least three limbs, one of them being an inhalation limb 24 for inspired gas, a second one being an expiration limb 25 for expired gas, a third one being a combined inspiration and expiration limb 26 for both inspired and expired gases. The inhalation limb 24 is connectable to the inspiration tube 18 and the expiration limb 25 is connectable to the expiration tube 19. The combined inspiration and expiration limb 26 of the branching unit 23 may be connectable by means of a patient tube 27 to the endotracheal tube 28 allowing the gas exchange with airways of the subject 4.

The inspiration gas flows from the reciprocating unit 6 through the ventilator tube 17, the CO2 remover 20, the first one-way valve 21 and the inspiration tube 18 of the patient circuit 2 to the branching unit 23 and further through the patient tube 27 and the endotracheal tube 28 to the lungs of the subject 4. The second one-way valve 22 on the expiratory tube 19 guides the gas flow direction to the inspiration tube 18 by closing the flow from the ventilator tube 17 through the expiration tube 19. Increasing the gas volume within the lungs increases the lung pressure due to the lung compliance. Once the inspiration stops and the expiration valve 9 opens relieving the bottle 14 pressure, the lung compliance pushes the alveolar gas through the endotracheal tube 28 and the patient tube 27 to the branching unit 23 and further through the expiration tube 19 and the ventilator tube 17 to fill the bellows 15.

The gas mixer 3 for delivering a fresh gas is connected to the breathing circuit 2 by means of a channel 35. The gas mixer 3 is used to offer the patient breathing gas. One or more gas supplies 5, 30, 31 is connected to the gas mixer 3. The gas supply 5 is for the air as described above, the gas supply 30 is for oxygen and the gas supply 31 is for nitrous oxide. The gas mixer comprises a valve 32 to select either the gas supply 31 for nitrous oxide or the gas supply 5 for air, a flow regulating valve 33 for regulating either nitrous oxide or air flow, a flow regulating valve 34 for regulating oxygen flow and an anesthetic agent supply 37 such as a vaporizer for supplying anesthetic agent to anesthetize the subject 4. The anesthetic agent supply 37 adds the inhalation agent into the gas mixture selected. Alternatively the anesthesia agent can be injected directly into the breathing circuit in a liquid form when it will be vaporized to the gas in the circuit or as a vapor.

The breathing circuit 2 may still be completed with a gas analyzer 39, which can be of a side-stream type as presented or a mainstream type. Side-stream analyzers take a sample gas flow for analysis from the breathing circuit 2. The gas analyzer 39, which is the mainstream type is analyzing the flowing gas directly in the breathing circuit 2 or in the patient tube 27.

The re-breathing circuit mode is used especially when delivering breathing gas with anesthetic agent or N02 to the subject as described above, but when delivering a breathing gas without anesthetic agent or N02 it is advantageous to exploit an open circuit mode in which case only partly same elements are exploited as used with the re-breathing circuit mode. Especially the CO2 remover 20 is not needed in the open circuit mode whereupon it can be bypassed when delivering the inspired gas flow from the gas supply 5 to the breathing circuit 2. A remarkable advantage is that a content of the CO2 remover 20 can be saved only for the re-breathing circuit mode use to remove CO2 exhaled by the subject. Also an inhaling resistance can be decreased by bypassing the CO2 remover 20. Further the inhaling resistance can be decreased by bypassing the reciprocating unit 6 in the open circuit mode.

In Figure 1 a selector valve 50 for the inspiration is connected to the first canal 8 of a ventilator 1 to guide the inspired gas flow from the gas supply 5 either through a first bypass passage 51 to the breathing circuit 2 downstream the CO2 remover 20 and the first one-way valve 21 and advantageously to the inspiration tube 18 bypassing both the CO2 remover 20 and the first one-way valve 21 or to the reciprocating unit 6 being in flow connection with the breathing circuit upstream the CO2 remover 20 whereupon the inspired gas flow guides respiratory movements of the subject through the CO2 remover 20, the first one-way valve 21 and the inspiration tube 18.

To guide the inspired gas flow the selector valve 50 for the inspiration in flow connection with the first bypass passage 51 is adapted to stop the inspiration gas flow either to the first bypass passage 51 in case the re-breathing circuit mode is selected and to open the inspiration gas flow to the reciprocating unit 6 or alternatively the selector valve 50 for the inspiration is adapted to stop the inspiration gas flow to the reciprocating unit 6 in case the open circuit mode is selected and to open the inspiration gas flow to the first bypass passage 51. The first bypass passage 51 can be connected to the breathing circuit 2 upstream or downstream the breathing circuit connection of the channel 35, but preferably the first bypass passage 51 is connected between the inhalation limb 24 of the branching unit 23 and the first one-way valve 21.

It is advantageous to connect one end of a second bypass passage 53 to a breathing circuit 2 upstream the second one-way valve 22, preferably between the second one-way valve 22 and the expiration limb 25 of the branching unit 23. Another end of of the second bypass passage 53 is connected to discharge the expiration gas flow to the second canal 10 between the reciprocating unit and the expiration valve 9. In flow connection with the second bypass passage 53 there is arranged a selector valve 52 for an expiration in the ventilator 1 to guide the expired gas flow in the expiration tube 19 of the breathing circuit 2 either through the second bypass passage 53 to between the reciprocating unit 6 and one of the expiration valve 9 and the scavenging valve 11 allowing an extra expired gas of the subject 4 to leave the breathing circuit 2 thus bypassing the reciprocating unit 6 making an expiration of the subject easier in the open circuit mode or through the second one-way valve 22 to the reciprocating unit 6 in the re-breathing circuit mode.

To guide the expired gas flow the selector valve 52 for the expiration is adapted to stop the expired gas flow either to the second bypass passage 53 in case the re-breathing circuit mode is selected and to open the expired gas flow to the reciprocating unit 6 or alternatively the selector valve 52 for the expiration is adapted to stop the expired gas flow to the reciprocating unit 6 in case the open circuit mode is selected and to open the expired gas flow through the second bypass passage 53 to between the reciprocating unit 6 and one of the expiration valve 9 and the scavenging valve 11. However, it is important to notice that this second bypass passage 53 is advantageous, but not absolutely necessary, because the most important thing is to arrange the first bypass passage 51 as explained hereinbefore. Thus the expiration gas flow can go towards the reciprocating unit 6 besides in case of the re-breathing circuit mode but also in case of the open circuit mode. The second bypass passage 53 for the expiration gas can be connected in the breathing circuit end also downstream the second one-way valve 22, but this connection would make the arrangement more complicated requiring extra valves to guide flows into the desired direction.

Figure 2 presents another embodiment for the reciprocating unit 6 useful in the anesthesia system. The reciprocating unit 6 comprises a long gas flow channel 41 functions differently from the one shown in Figure 1. The channel 41 is part of a continuous flow pathway between said gas supply 5 and said breathing circuit 2. The separation of the ventilator gas and the breathing gas is made with a gas gradient reciprocating in the gas flow channel 41. In this the receiving information indicative of the measured gas flows the gas supply 5 pushes the inspiration gas towards the ventilator 1 and through the flow control valve 7 to control the inspiration gas flow and to the channel 41. This will push the prevailing gas from the channel 41 further to the inspiration tube 18 of the breathing circuit 2 and further to the subject 4. During the expiration the expiration valve 9 is opened releasing the pressure of the channel 41 and the breathing circuit 2. The lung compliance drives the gas out from the lungs through expiration tube 19 to the channel 41. This pushes the gas loaded into the ventilator end of the channel 41 during the inspiration out from the channel 41 through the expiration valve 9 of the ventilator 1. Would the fresh gas stream into the breathing circuit 2 exceed the one removed, the excess flows through the channel 41 of the reciprocating unit 6 and gets also scavenged through the expiration valve 9.

Also in Figure 2 the selector valve 50 for the inspiration is arranged to guide the inspired gas flow from the gas supply 5 either in the open circuit mode through the first bypass passage 51 to the breathing circuit 2 downstream the CO2 remover 20 and the first one-way valve 21 to the inspiration tube 18 bypassing both the CO2 remover 20 and the first one-way valve 21 or in the re-breathing circuit mode to the reciprocating unit 6 being in flow contact with the breathing circuit upstream the CO2 remover 20 whereupon the inspired gas flow guides respiratory movements of the subject through the CO2 remover 20, the first one-way valve 21 and the inspiration tube 18. The selector valve 50 is arranged both in Figure 1 and 2 upstream the reciprocating unit 6 in which case it may be part of the ventilator 1.

To guide the inspired gas flow in Figure 1 the selector valve 50 for the inspiration is adapted to stop the inspired gas flow either to the first bypass passage 51 in case the re-breathing circuit mode is selected and to open the inspired gas flow to the reciprocating unit 6 or alternatively the selector valve 50 for the inspiration is adapted to stop the inspired gas flow to the reciprocating unit 6 in case the open circuit mode is selected and to open the inspired gas flow to the first bypass passage 51. The first bypass passage 51 can be connected to the breathing circuit 2 upstream or downstream a breathing circuit connection of the channel 35, but preferably the first bypass passage 51 is connected between the inhalation limb 24 of the branching unit 23 and the first one-way valve 21.

In Figure 2, just as with Figure 1 embodiment, the selector valve 52 for the expiration with the second bypass passage 53 is arranged to make the expiration of the subject easier. The selector valve 52 for the expiration connecting an end of the second bypass passage 53 to the breathing circuit 2is arranged between the second one-way valve 22 and the expiration limb 25 of the branching unit 23 to guide the expired gas flow in the expiration tube 19 of the breathing circuit 2 either through the second bypass passage 53 to the second canal 10 downstream the reciprocating unit 6 which may be between the reciprocating unit 6 and the expiration valve 9 allowing an extra expired gas of the subject 4 to leave the breathing circuit 2 thus bypassing the reciprocating unit 6 in the open circuit mode or through the second one-way valve 22 towards the reciprocating unit 6 in the re-breathing circuit mode.

To guide the expired gas flow the selector valve 52 for the expiration is adapted to stop the expired gas flow either to the second bypass passage 53 in case the re-breathing circuit mode is selected and to open the expired gas flow towards the reciprocating unit 6 or alternatively the selector valve 52 for the expiration is adapted to stop the expired gas flow towards the reciprocating unit 6 in case the open circuit mode is selected and to open the expired gas flow through the second bypass passage 53 to between the reciprocating unit 6 and the expiration valve 9. Just as with Figure 1 also with Figure 2 it is important to notice that this second bypass passage 53 is advantageous but not absolutely necessary, because the most important thing is to arrange the first bypass passage 51 as explained hereinbefore. Thus the expiration gas flow can go towards the reciprocating unit 6 besides in case of the re-breathing mode but also in case of the open circuit mode. The selector valve 52 for the expiration as well as the second bypass passage end in the breathing circuit 2 can also locate downstream the second one-way valve 22, but this arrangement would make the arrangement more complicated requiring extra valves to guide flows into the desired direction.

Otherwise the embodiment of Figure 2 is used on the same principles as described above in connection to Figure 1. The gas mixer 3 can be identical to Figure 1.

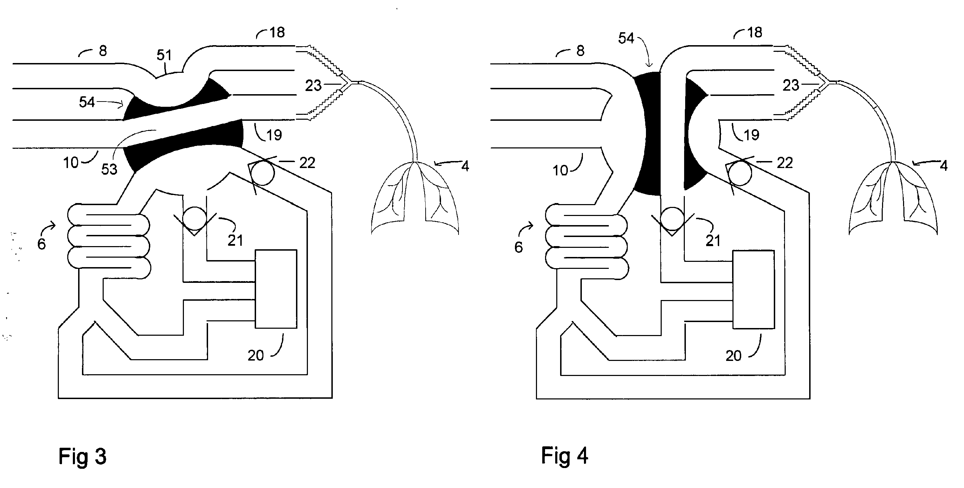

Figures 3, 4, 5 and 6 shows a solution which can be exploited in Figures 1 and 2 to replace the selector valve 50 for the inspiration and the selector valve 52 for the expiration by a single combined selector valve 54 for both the inspiration and expiration being in flow connection with the first bypass passage 51 and the second bypass passage 53. According to an option this selector valve 54 is arranged to supply the inspiration gas flow to the breathing circuit 2 bypassing the reciprocating unit 6 and also an option to arrange simultaneously the expiration gas flow of the breathing circuit to be discharged bypassing the reciprocating unit. The selector valve 54 for both the inspiration and expiration in a first position enables isolation and detaching the CO2 remover and the reciprocating unit 6 when the open circuit mode is selected and vice versa in a second position the combined selector valve 54 enables connecting the CO2 remover and the reciprocating unit 6 when the re-breathing circuit mode is selected.

In Figures 3 and 4 the selector valve 54 for both the inspiration and expiration is a rotary valve, which can be rotated from the first position to the second position. In Figure 3 the open circuit mode is selected where the selector valve 54 for both the inspiration and expiration is in the first position and thus stopping the inspired gas flow from going through the reciprocating unit 6 and the CO2 remover 20 and the expired gas flow from going towards the reciprocating unit 6, but bypassing them. The inspiration gas flow is coming along the first canal 8 to the selector valve 54 for both the inspiration and expiration, which in this first position directs the flow through the first bypass passage 51 to the inspiration tube 18 and the branching unit 23 to the subject 4 bypassing both the reciprocating unit 6 and the CO2 remover 20. When expiring the expired gas is led through the branching unit 23 and the expiration tube 19 to the selector valve 54 for both the inspiration and expiration which in the first position directs the expired gas through the second bypass passage 53 to the second canal 10 for the expired gas bypassing the reciprocating unit 6.

Figure 4 representing a situation where the re-breathing circuit mode is selected the selector valve 54 for both the inspiration and expiration is in the second position directing the inspiration gas flow to the reciprocating unit 6 whereupon the gas flows in the breathing circuit through the CO2 remover 20, the first one-way valve 21, the inspiration tube 18 and the branching unit 23 to the subject 4. During the expiration phase the expired gas is flowing through the branching unit 23, the expiration tube 19 and the second one-way valve 22 towards the reciprocating unit 6 to discharge the expired gas in the second canal 10.

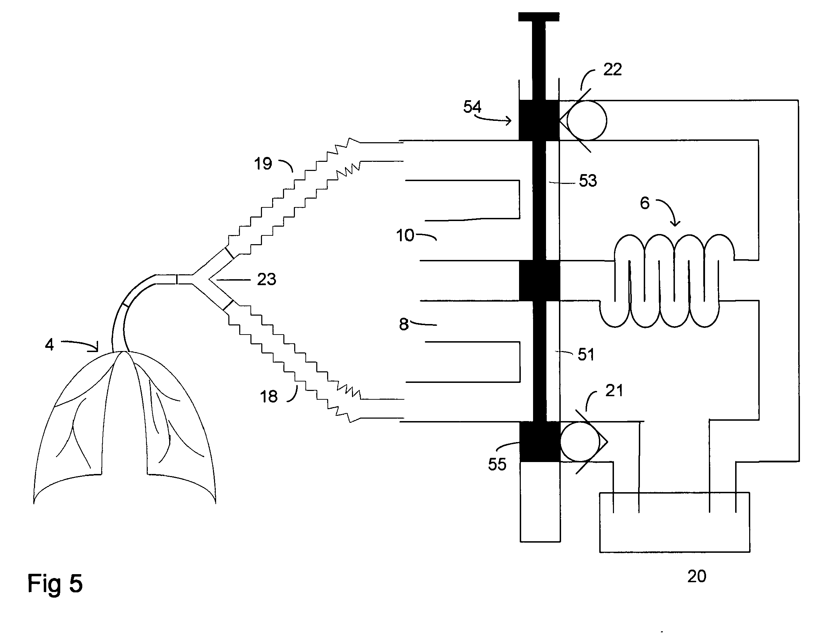

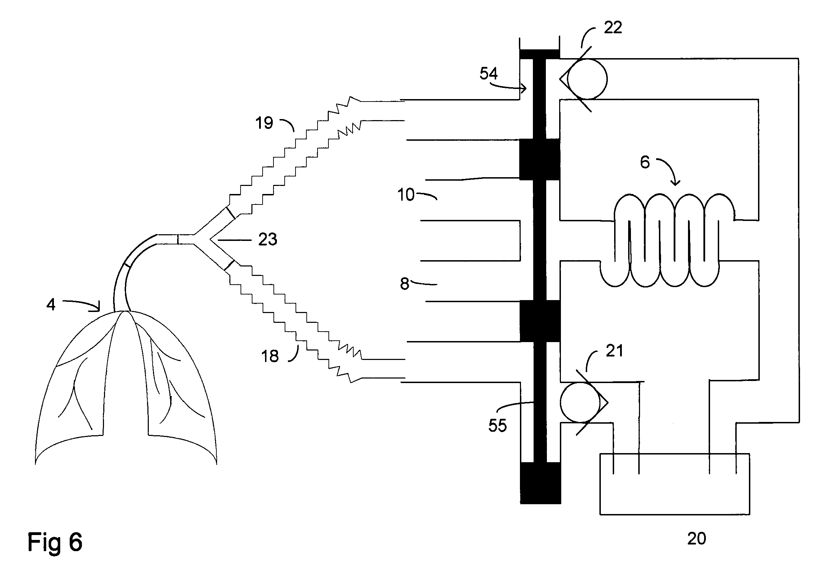

The selector valve 54 for both the inspiration and expiration can be also a slide valve as shown in Figure 5 and 6 having a chance to select between the first position and the second position. A slide 55 can slide up and down to find either the first position or the second position. In the first position as shown in Figure 5 the slide 55 closes the inspiration gas flow towards the reciprocating unit 6 and through the CO2 remover 20. Also the expired gas flow towards the reciprocating unit 6 is closed. The inspired gas flow is coming along the first canal 8 to the selector valve 54 for both the inspiration and expiration, which in this first position directs the flow through the first bypass passage 51 to the inspiration tube 18 and further to the branching unit 23 and to the subject 4 bypassing both the reciprocating unit 6 and the CO2 remover 20. When expiring the expired gas is conducted through the branching unit 23 and the expiration tube 19 to the selector valve 54 for both the inspiration and expiration, which in the first position directs the expired gas through the second bypass passage 53 to the second canal 10 for the expired gas bypassing the reciprocating unit 6.

The re-breathing circuit mode is selected in Figure 6 where the theselector valve 54 for the inspiration and expiration is in the second position directing the inspiration gas flow towards the reciprocating unit 6 and through the CO2 remover 20 to the first one-way valve 21 guiding the inspired gas flow through the inspiration tube 18 and the branching unit 23 to the subject 4. During the expiration phase the expired gas is flowing from the subject 4 through the branching unit 23, the expiration tube 19, the second one-way valve 22 towards the reciprocating unit 6 to discharge the expired gas through the second canal 10.

Isolation and detaching the CO2 remover 20 and the reciprocating unit 6, enables the open circuit mode use also in situations when the CO2 remover 20 and the reciprocating unit 6 are not available or needed. On this account solution of two isolated breathing circuit systems in the same device increases patient safety and efficiency of utilization. For example, in situations when the subject needs the respiratory care after the anesthesia, the CO2 remover 20 and the reciprocating unit 6 can be removed and maintain for next anesthesia, if the open circuit mode is used, and after the maintenance it can again be attached to the system without contamination.

The removable CO2 remover 20 and reciprocating unit 6 allow a development of a block solution for the re-breath circuit, and this way allows practicable disposableness. It also facilitates regular cleaning and maintenance of the system, even when the ventilator 1 is in use and when a single selector valve 54 for both the inspiration and expiration such as the rotary or slide valve is in use. The re-breathing circuit is connected in the system by three connections as shown in Figures 3-6, which are close to each other making the disconnection of the CO2 remover 20 and the reciprocating unit 6 easy, quick and safe.

The isolated re-breathing system decreases a demand of the reciprocating unit 6 and the CO2 remover 20 when the open breathing system is used. It also decreases a need for cleaning and maintenance of the re-breathing system. This cuts down hospital expenses, because those elements need to replace for new ones less frequently.

Changing between the open and re-breath circuit system, and vice versa, is fast and is assumed to be safe, and it allows optimal breathing circuit use for every anesthesia type.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. An arrangement for supplying a breathing gas for a respiration comprising:
a reciprocating unit (6) receiving an inspiration gas flow adapted to control respiratory movements;
a gas mixer (3) for supplying a fresh gas flow; and
a breathing circuit (2) for conducting an expiration gas flow to said reciprocating unit (6) and for conducting the fresh gas flow from said gas mixer (3) for the respiration and for conducting the gas flow from said reciprocating unit (6) for an inspiration, said breathing circuit comprising a CO2 remover (20) for removing carbon dioxide from the gas,
**characterized in that** said arrangement also comprising a first bypass passage (51) bypassing said reciprocating unit (6) and connecting said breathing circuit (2) to the inspiration gas flow upstream said reciprocating unit (6).

2. The arrangement according to claim 1, **characterized in that** said breathing circuit (2) also comprising a first one-way valve (21) for the inspired gas, an inspiration tube (18) and an inhalation limb (24) of a branching unit (23) allowing the gas coming from the CO2 remover (20) to flow through said first one-way valve, said inspiration tube (18) and said inhalation limb (24) for a subject breathing.

3. The arrangement according to claim 1, **characterized in that** upstream said reciprocating unit (6) is provided a selector valve (50) for the inspiration to connect the inspiration gas flow either to the reciprocating unit (6) or through said first bypass passage (51).

4. The arrangement according to claim 3, **characterized in that** said selector valve (50) for the inspiration is connected to a first canal (8) supplying the inspiration gas flow to said reciprocating unit (6), which first canal is upstream the reciprocating unit.

5. The arrangement according to claim 2, **characterized in that** said first bypass passage (51) is connected to the breathing circuit (2) downstream said first one-way valve (21) in which case the flow through said first bypass passage is bypassing said CO2 remover (20) while flowing towards an inhalation limb (24) of a branching unit (23).

6. The arrangement according to claim 1, further comprising a second bypass passage (53) having one end connectable to said breathing circuit (2) for removing the expired gas and allowing the expired gas to bypass said reciprocating unit (6).

7. The arrangement according to claim 6, **characterized in that** another end of said second bypass passage (53) is connected to a second canal (10), which second canal extends between said reciprocating unit (6) and an expiration valve (9).

8. The arrangement according to claim 6, **characterized in that** the expiration gas flow either through said second bypass passage (53) or towards said reciprocating unit (6) is guided by a selector valve (52) for the expiration being in flow connection with said second bypass passage.

9. The arrangement according to claim 6, **characterized in that** said breathing circuit (2) further comprising a second one-way valve (22) for the expired gas, an expiration tube (19), an expiration limb (25) of a branching unit (23) allowing the expired gas coming from a subject to flow through said expiration limb (25) and said expiration tube (19) towards a selector valve (52) locating between said expiration limb of said branching unit (23) and said second one-way valve (22).

10. The arrangement according to claim 6, **characterized in that** both said first bypass passage (51) and said second bypass passage (53) are equipped with a single selector valve (54) for both the inspiration and expiration being capable of bypassing in a first position said reciprocating unit (6) and in a second position guiding both the inspired gas and the expired gas towards said reciprocating unit (6).

11. The arrangement according to claim 1, **characterized in that** said reciprocating unit (6) comprising either a bottle (14) and a bellows (15) within said bottle being adapted to separate a gas flow upstream and downstream said reciprocating unit or a gas flow channel (41) being part of a continuous flow pathway upstream said breathing circuit (2).

12. A method for supplying a breathing gas for a respiration comprising:
supplying an inspiration gas flow to a reciprocating unit (6) to control respiratory movements;
supplying a fresh gas flow to a breathing circuit (2); and
conducting along a breathing circuit (2) an expiration gas flow to said reciprocating unit (6) and the fresh gas flow for the respiration and a gas flow from said reciprocating unit (6) for inspiration, and removing carbon dioxide from the gas flowing in said breathing circuit,
**characterized in that** said method also comprising an option to supply the inspiration gas flow to said breathing circuit (2) bypassing said reciprocating unit (6).

13. The method according to claim 12, **characterized in that** carbon dioxide is removed by a CO2 remover (20) and said option to supply the inspiration gas flow to said breathing circuit (2) also is adapted to bypass said CO2 remover.

14. The method according to claim 12, **characterized in that** said method also comprising an option to allow the expiration gas flow of said breathing circuit (2) to be discharged when desired bypassing said reciprocating unit (6).

15. The method according to claim 12, **characterized in that** said option to supply the inspiration gas flow to said breathing circuit (2) bypassing said reciprocating unit (6) also includes an option to arrange simultaneously the expiration gas flow of said breathing circuit (2) to be discharged bypassing said reciprocating unit (6).
